# EUROPEAN PATENT SPECIFICATION

(11) **EP 0 982 042 B1**
(45) Date of publication and mention of the grant of the patent: **26.11.2003**
(21) Application number: 98306923.8
(22) Date of filing: 28.08.1998
(51) Int. Cl.: A61M 16/00

(54) **Medical face mask**
Medizinische Gesichtsmaske
Masque facial médical

(43) Date of publication of application: 01.03.2000
(73) Proprietor: Hellings, Deborah, Tallahassee, FL 32315 (US)
(72) Inventor: Hellings, Deborah, Tallahassee, FL 32315 (US)
(74) Representative: Moore, Derek

(56) References cited:
- EP-A- 0 288 937
- WO-A-97/00092
- BE-A- 456 433
- US-A- 3 695 264

## Description

### Field of the Invention

The present invention relates to a medical face mask, in particular to an anaesthesia and/or respiratory face mask.

### Description of the Prior Art

Face masks of this type are known. During surgery, a patient is usually placed under anaesthesia. A face mask is placed over face of the patient and in particular the patient's nose and mouth and anaesthetic gases are introduced into the area between the mask and the patient's face. The gases are inhaled by the patient, thereby causing the patient to be anaesthetized.

Similar types of masks can be used by patients who have need of a respirator. Such patients include those with breathing difficulties such as asthmatics and people needing gaseous pain killers, for example women in childbirth. The air and/or medicinal gases are introduced into the mask, which are breathed in by the patient, thereby having an effect on the patient.

Known anaesthesia and respirator masks have several problems. The mask has to be securely held against the patients face so that an area around the periphery of the mask remains in close contact with the patient's face. A substantially gas tight seal between the mask and the patient's face is thereby made. The area around the periphery of the mask often comprises a deformable ring of material such as rubber which can be compressed to form the seal.

This close contact between the mask and the patient's face causes pressure on the area of the face including the bridge of the nose of the patient and can cause discomfort and irritation. This in turn can cause bruising and pressure damage to the patient's face, which in turn can lead to nerve damage which may cause paralysis of the patient's face.

Further due to the oval shape of known masks, such masks often extend upwards beyond the bridge of the nose of the wearer and into the eye region. Pressure in the eye region of the patient can lead to extreme discomfort and possible eye damage. Especially when the mask is a respirator type mask being used personally by a patient, rather than be applied to the patent by medical staff, then the discomfort often leads to the patient removing the mask from his face. If the mask is removed, there can be diminished or reduced introduction of medicinal gases and/or air to the patient which will reduced in decreased efficiency of treatment of that patient due to the application of incorrect doses of gaseous drugs. The disadvantages of known masks is further emphasised by the fact that such masks are not designed to accommodate the different facial shapes of possible wearers.

US 4799477 discusses a mask where there is close contact with the face and the apex of the mask firmly rests against the bridge of the nose. Similarly US 3721238, US 3815596, DE 1104122 and US 2625155 disclose such devices, which have a shape which is substantially oval. US 2765788 discloses a shaped face mask but as in the other cited art, the sides of the mask are wider than the top of the mask this shaping will again result in a mask which has increased pressure on the bridge of the nose of a patient, leading to discomfort.

Further to maintain the mask in position it is necessary to have structures such as straps passing around the back of the head of the patient. These straps again press against the side of the patient's face which again can result in nerve damage which ultimately can lead to discomfort and paralysis of the patient's face. US 3815596 discusses strap arrangements for holding an anaesthetic mask in position but in this case, the straps lie against the patient's face. US 4337767 and US Design 293613 discuss hook means for holding straps to a face mask but in these cases, the hook means do not extend far outwards from the mask to prevent the straps from pressing on the side of a patient's face .

### Summary of the Invention

The aim of the present invention is to eliminate the pressure that is exerted on a patient's face by the edges of the mask. Also, the mask of the present invention has a support structure to hold the mask in place which does not press on the sides of the patient's face.

According to the present invention there is provided a face mask 1 for medical purposes comprising:
- a shaped base member 2 having a front wall portion 3 and a side wall 5 which defines an open portion 6 enabling the base member to be placed over a patient's face,
- said shaped base member 2 having sealing means 7 around a peripheral edge of the side wall 5,
- characterised in that the base member, when viewed face on, is substantially heart shaped and when the mask is placed over the patient's face, the upper part of the mask 9 is wider than the lower part of the mask 11, and the indentation 9a in the upper region 9 of the heart shaped mask extends towards a region of the patient's nose below the bridge of said patient's nose, thereby eliminating contact between the face mask and the bridge of the patient's nose.

In a first embodiment of the invention, the face mask is of a synthetic polymeric resin and preferably, the mask is of a disposable type. The disposability of the face mask has the added benefit that there is reduced likelihood of cross contamination between patients. However, it is possible to have a mask of other materials such as rubber or even glass, which can be sterilised if needed between patients.

According to a further embodiment of the invention, it is preferable that the mask is of a transparent type. This is to enable medical staff to monitor the patient more easily as in certain circumstances, the patient may vomit, either due to their illness or because they have reacted to the drugs that are being administered to them by way of the mask. If the vomit is not removed from the patient's face there is the risk if it being inhaled and the patient may choke. If medical staff can see that this has occurred through the transparent mask, they can then attend to cleaning the patient. However, it is envisaged that non transparent masks may be used.

The front wall of the mask is attached to means such as tubing, to enable gas and/or medication to be introduced into the mask.

The sealing means around the side wall of the mask is preferably of pliable material and in a preferred embodiment of the invention, the mask can be inflated thereby providing a softer surface against the patient's face.

The mask is held on the patient's head by a head strap which runs from the front of the mask to the back of the patient's head. At the front of the mask there are a series of projections, typically rods which extend from the front surface of the mask. These rods have means for connecting straps which are to extend around the back of the patient's head to the rods. The rods are of a length which enables the rods to hold the straps away from the patient's face. As the straps do not press against the patient's face the risk of bruising and nerve damage to the patient is minimised.

### Brief Description of the Drawings

Figure 1 is a top elevational view of a mask of the present invention.
Figure 2 is a rear elevational view of a mask of the present invention.
Figure 3 is a side elevational view of a mask of the present invention.
Figure 4 is a view of the strap of the present invention.
Figure 5 is a view of a mask according to the prior art.
Figure 6 is a view of the mask according to the invention showing an indentation which eliminates contact of the mask with the bridge of the patient's nose and of equal or greater importance, eliminate's contact with the patient's ocular area.

### Description of the preferred embodiments of the Invention

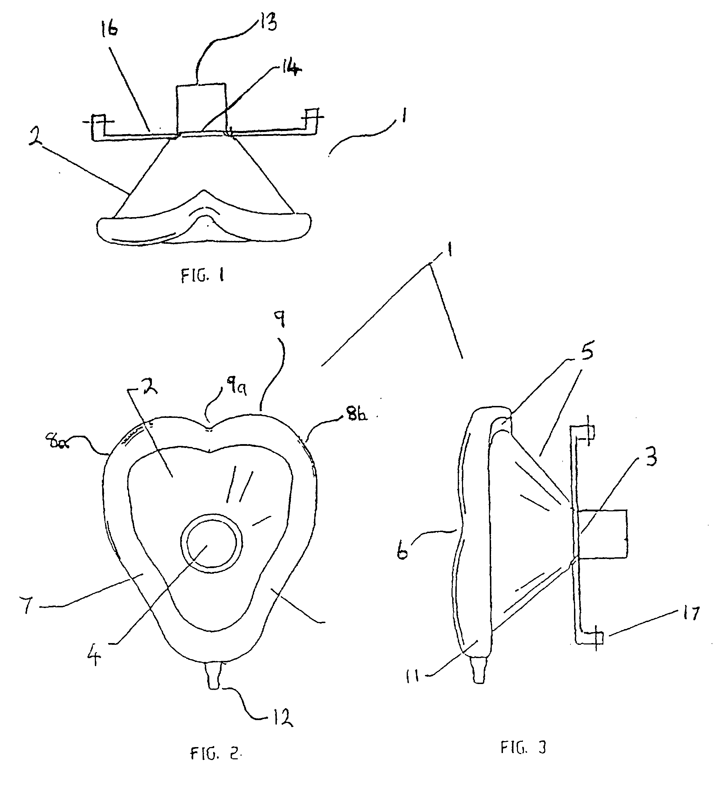

Figs. 1 - 3 represent various views of the face mask 1 of the present invention, which preferably is an anaesthesia/respiratory mask. The mask 1 comprises a transparent polymeric resin base member 2, which is concaved towards a patients face (not illustrated). The concave or cup-shaped mask 1 has a front wall 3 having an aperture 4, an encompassing side wall 5, and an opened bottom portion 6 for receiving the nose and mouth of the patient. In an alternative arrangement, base member 2, is not of a transparent material and it may be of an opaque material such as rubber.

The encompassing side wall 5 has a peripheral edge comprising bottom portion 11, top portion 9, and side portions 8a and 8b. The bottom portion 11 is substantially in the shape of a horseshoe while the shape of the top portion 9 is of a serial double arch, each of the arches being separated by an indentation 9a. There is a constant radius of curvature for each arch. However, it is envisaged, that the radius of curvature of each arch may be different, for example if a face mask is tailor made for a particular patient. The side portions 8a and 8b bridge both ends of bottom portion 11 to their respective ends of top portion 9. The whole periphery of the mask forms substantially a heart shape. This shape provides for the mask to curve around the nasal bridge and the oral tissues in order to confine the nasus and oris of a patient.

Affixed to the edge of the encompassing side all is a soft pliable sealing material 7. The sealing material 7 is preferably inflatable, however non-inflatable sealing means may be used. A protruding nipple 12 is located at the bottom of the sealing material 7. An air pump (not illustrated) can be attached to this nipple 12 in order to enable for the inflation of the soft pliable material 11.

A portal 13 is located on the mask and is used for the induction of a variety of gases, such as oxygen, nitrogen and other anaesthetic gases or the like. The portal 13 includes a protruding hollow cylindrical tube which is attached to the aperture 4 of the front wall portion 3.

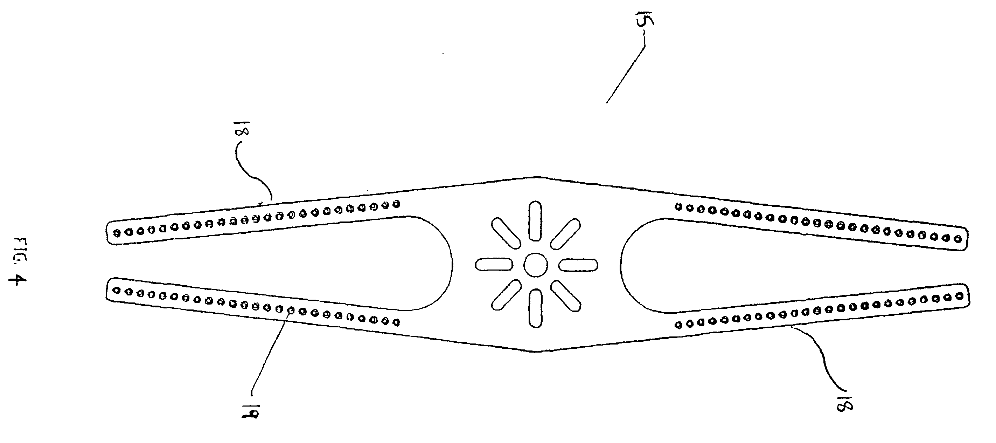

A head strap plate 14 is adapted to be removably fitted on the portal 13. Attached to and protruding outwardly from head strap plate 14 are a plurality of elongated rods 16. As illustrated in the figures, the rods are substantially perpendicular to the portal and extend radially from the portal 13. Each rod has a length of at least 2 to 6 cms and preferably 4 to 5 cms. Located on the outer ends of each rod is a hook 17. These hooks receive and maintain a strap (illustrated and discussed in further detail in fig. 4) that can be used in combination with the mask. The arrangement and design of the rods and hooks attached to the portal provides for the strap not to contact the patient when the mask is in utilization. Located at the outer ends of each hook 17 are ring shaped strap seats (illustrated in figs 1 and 3 but not labelled). These straps seats maintain the strap in a fixed position. These strap seats provide a resting means for the strap and also provide a stop so that the strap does not travel downward on the hook.

Typically there would be four elongated rods that the affixed to the heat strap plate. However, more or less than four rods could be used, depending on the requirements needed to provide a mask with an adequate fit. For example it may be that for a baby or a child, then more than four rods would be used, firstly to provide more points for strap attachment as more straps may be needed to stop the mask from being dislodged due to movement of the child. A second reason for using more rods and strap attachments is to increase the load distribution of the mask against the face. These rods are transverse from the axis of the portal and are substantially 90 degrees from one another.

Fig. 4 illustrates a strap 15 which can be attached to the hooks on the mask. The strap 15, preferably is a spider shaped design having a central body (not labelled) and a plurality of prongs 18. The number of prongs would correspond to the number of rods which are located on the head strap plate. The strap is made of a durable elastic material, such as rubber. Each prong 18 is equipped with several holes 19. The hooks on the strap receive the holes on the prong. This design provides for the holes to act as an adjusting means for the strap.

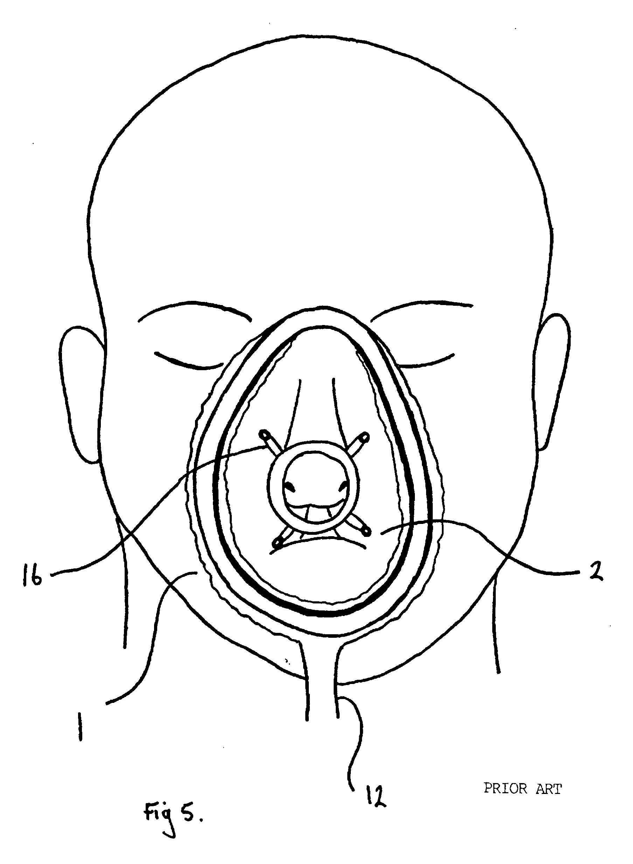

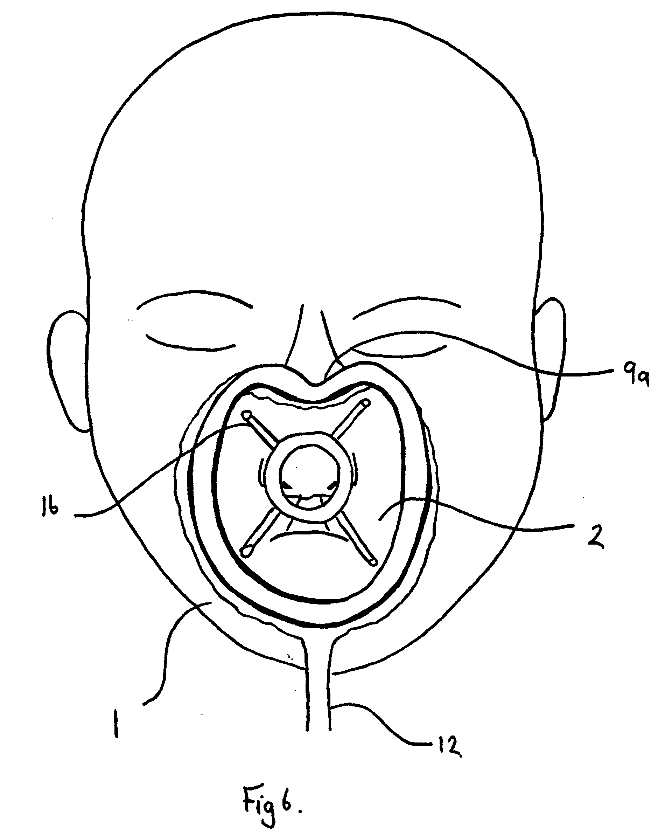

In order to use the mask, it is first placed on the patients face. A mask according to the invention, when placed on a patient's face is shown in figure 6, while a mask according to the prior art is shown in figure 5. This can be seen in figure 6. Air is inducted through the nipple to inflate the sealing material. Once so inflated, the sealing material will provide a pneumatic seal around the patient's nose and mouth. An anaesthesia gas inlet line and exhaust gas outlet line (neither shown) are attached to the portal to provide for a completed anaesthetic circuit. Gases enter and exit the mask chamber through the portal and the aperture.

In order to attach the strap to the mask, the strap is first placed so that the central body is located against the back of the patient's head. Then each of the prongs are placed around the patient's head and are positioned so that a particular hole is aligned with a hook on the head strap plate. The choice of the particular hole will be dependent on the size of the patient's head. Each prong is then fitted onto the hook so that the hook protrudes through it selected hole. Each prong will come to rest on a ring seat thereby provided a secure fit of the mask to the patient.

Due to the substantially perpendicular placement of the rods with respect to the portal, the rod extended size of at least 4 to 5 cms, the hooks being perpendicular to the rods, and the placement of the seat ring at the hooks outer end, the prongs of the strap will not communicate with the patient's face.

Figures 5 and six show respectively a face mask according to the prior art and a face mask according to the invention. It is clear from figure 5 that the mask of the prior art sits firmly on the bridge of the patient's nose and intrudes on the ocular area of the patient. Figure 6 shows that a face mask according to the present invention, which is of a substantially heart shape, provides a mask where there is no pressure on the bridge of the patient's nose and no pressure on the patient's ocular area, so the discomfort associated with known face masks is eliminated. The indentation 9a provides a recessed area at the top of the mask which extends below the bridge of the nose and towards the central region and the tip area of the nose, ie the nostril area of the nose. In an a usual situation, the indentation extends to an area substantially midway between the bridge of the nose and the tip of the nose. However, depending on the facial type of the patient, the indentation may extend down as far as the tip area of the nose. In the description, it is intended that bridge of the nose refers to that part of the nose, which is in proximity to a person's forehead and extends to a distance of approximately lcm and up to 2 cms from the forehead, depending on facial characteristics of a person. The bridge is formed from bone whilst the remaining part of the nose is shaped from cartilaginous material.

While the invention has been particularly shown and described with reference to an embodiment thereof, it will be understood by those skilled in the art, that various changes in form and detail may be made without departing from the scope of the invention.

## Claims

1. A face mask (1) for medical purposes comprising:
- a shaped base member (2) having a front wall portion (3) and a side wall (5) which defines an open portion (6) enabling the base member to be placed over a patient's face,
- said shaped base member (2) having sealing means (7) around a peripheral edge of the side wall (5),
- **characterised in that** the base member, when viewed face on, is substantially heart shaped and when the mask is placed over the patient's face, the upper part of the mask (9) is wider than the lower part of the mask (11), and the indentation (9a) in the upper region (9) of the heart shaped mask extends towards a region of the patient's nose below the bridge of said patient's nose, thereby eliminating contact between the face mask and the bridge of the patient's nose.

2. A face mask according to claim 1, **characterised in that** the base member (2) is made of a substantially transparent material.

3. A face mask according to claim 2, **characterised in that** the base member (2) is made of polymeric material.

4. A face mask according to any preceding claim, **characterised in that** the sealing means (7) comprises an inflatable member.

5. A face mask according to any preceding claims having projections (16) protruding radially from the front wall portion (3) of the mask.

6. A face mask according to claim 5, **characterised in that** the projections (16) are rod shaped members.

7. A face mask according to claim 5 or claim 6, **characterised in that** the projections are between 4 and 5cms in length.

8. A face mask according to claims 5, 6 or 7, **characterised in that** the projections (16) have attachment means (17) to which a head strap (15) means for the mask can be detachably secured.

9. A face mask according to claim 8 **characterised in that** the head strap means is adjustable in length.

10. A face mask according to any preceding claim, **characterised in that** the face mask is disposable.

## Patentansprüche

1. Eine Gesichtsmaske (1) für medizinische Zwecke mit:
- einem geformten Trägerelement (2) mit einem vorderen Wandteil (3) und einer Seitenwand (5), die einen offenen Teil (6) umschreibt, durch den das Trägerelement über das Gesicht des Patienten platziert werden kann,
- wobei das Trägerelement (2) entlang des Umfanges der Seitenwand (5) ein Dichtungsmittel (7) trägt,
- **dadurch gekennzeichnet, dass** das Trägerelement von vorne betrachtet im wesentlichen herzförmig ist und dass, wenn die Maske über dem Gesicht des Patienten platziert wurde, der obere Teil der Maske (9) breiter als der untere Teil der Maske (11) ist und dass sich die Einbuchtung (9a) im oberen Bereich (9) der herzförmigen Maske in Richtung eines Teils der Nase des Patienten erstreckt, der unterhalb der Brücke der Nase liegt, so dass ein Kontakt zwischen der Gesichtsmaske und der Brücke der Nase des Patienten vermieden wird.

2. Eine Gesichtsmaske nach Anspruch 1, **dadurch gekennzeichnet, dass** das Trägerelement (2) aus einem im Wesentlichen durchsichtigen Material besteht

3. Eine Gesichtsmaske nach Anspruch 2, **dadurch gekennzeichnet, dass** das Trägerelement (2) aus einem polymeren Material besteht.

4. Eine Gesichtsmaske nach einem der vorangegangenen Ansprüche, **dadurch gekennzeichnet, dass** das Dichtungsmittel (7) ein aufblasbares Element enthält

5. Eine Gesichtsmaske nach einem der vorangegangenen Ansprüche mit Fortsätzen (16), die radial vom vorderen Wandteil (3) der Maske ausgehen.

6. Eine Gesichtsmaske nach Anspruch 5, **dadurch gekennzeichnet, dass** die Fortsätze (16) aus stabförmigen Elementen bestehen.

7. Eine Gesichtsmaske nach Anspruch 5 oder Anspruch 6, **dadurch gekennzeichnet, dass** die Fortsätze zwischen 4 und 5 cm lang sind.

8. Eine Gesichtsmaske nach den Ansprüchen 5, 6 oder 7, **dadurch gekennzeichnet, dass** die Fortsätze (16) mit einem Befestigungsmittel (17) versehen sind, an dem separierbar ein Kopfgurt (15) für die Maske angebracht werden kann.

9. Eine Gesichtsmaske nach Anspruch 8, **dadurch gekennzeichnet, dass** der Kopfgurt in seiner Länge verstellbar ist.

10. Eine Gesichtsmaske nach einem der vorangegangenen Ansprüche, **dadurch gekennzeichnet, dass** es sich bei der Maske um einen Einwegartikel handelt.

## Revendications

1. Masque facial (1) pour applications médicales comprenant :
- un élément de base façonné (2) ayant une partie de paroi avant (3) et une paroi latérale (5) qui définit une partie ouverte (6) permettant à l'élément de base d'être placé sur le visage d'un patient,
- ledit élément de base façonné (2) comportant des moyens d'étanchéité (7) autour d'un bord périphérique de la paroi latérale (5),
- **caractérisé en ce que** l'élément de base, quand il est vu de face, est sensiblement en forme de coeur et quand le masque est placé sur le visage du patient, la partie supérieure du masque (9) est plus large que la partie inférieure du masque (11), et la découpe (9a) pratiquée dans la région supérieure (9) du masque en forme de coeur s'étend vers une région du nez du patient sous l'arête dudit nez du patient, éliminant de la sorte le contact entre le masque facial et l'arête du nez du patient.

2. Masque facial selon la revendication 1, **caractérisé en ce que** l'élément de base (2) est fabriqué en matériau sensiblement transparent.

3. Masque facial selon la revendication 2, **caractérisé en ce que** l'élément de base (2) est fabriqué en matériau polymère.

4. Masque facial selon l'une quelconque des revendications précédentes, **caractérisé en ce que** le moyen d'étanchéité (7) comprend un élément gonflable.

5. Masque facial selon l'une quelconque des revendications précédentes, comportant des saillies (16) dépassant dans le sens radial de la partie de paroi avant (3) du masque.

6. Masque facial selon la revendication 5, **caractérisé en ce que** les saillies (16) sont des éléments en forme de tiges.

7. Masque facial selon la revendication 5 ou la revendication 6, **caractérisé en ce que** les saillies mesurent entre 4 et 5 cm de long.

8. Masque facial selon les revendications 5, 6 ou 7, **caractérisé en ce que** les saillies (16) comportent des moyens de fixation (17) auxquels un moyen de sangle de tête (15) du masque peut être fixé de façon détachable.

9. Masque facial selon la revendication 8, **caractérisé en ce que** le moyen de sangle de tête est réglable en longueur.

10. Masque facial selon l'une quelconque des revendications précédentes, **caractérisé en ce que** le masque facial est jetable.
